# EUROPEAN PATENT SPECIFICATION

(11) **EP 3 325 234 B1**
(45) Date of publication and mention of the grant of the patent: **09.09.2020**
(21) Application number: 16828363.8
(22) Date of filing: 18.07.2016
(51) Int. Cl.: B25J 19/00, A61B 46/10, A61B 34/30, A61B 17/00

(54) **PROTECTIVE DRAPE FOR ROBOTIC SYSTEMS**
SCHUTZTUCH FÜR ROBOTERSYSTEME
CHAMP DE PROTECTION POUR SYSTÈMES ROBOTIQUES

(30) Priority: 23.07.2015 US 201562196073 P
(43) Date of publication of application: 30.05.2018
(73) Proprietor: Think Surgical, Inc., Fremont, CA 94538 (US)
(72) Inventor: BONNY, Daniel, P., Fremont, CA 94538 (US); ZUHARS, Joel, Fremont, CA 94538 (US); TABANDEH, Saleh, Fremont, CA 94538 (US); PACK, Timothy, Fremont, CA 94538 (US); HANSON, Randall, Fremont, CA 94538 (US); HOPPE, Michael, E., Fremont, CA 94538 (US); NETRAVALI, Nathan, A., Fremont, CA 94538 (US); GOLDSTEIN, Avery, A., Birmingham, MI 48009 (US)
(74) Representative: Müller Schupfner & Partner Patent- und Rechtsanwaltspartnerschaft mbB
(86) International application number: PCT/US2016/042786
(87) International publication number: WO 2017/015207

(56) References cited:
- WO-A2-2007/143162
- DE-A1-102012 217 445
- US-A- 4 799 779
- US-A- 5 122 904
- US-A- 5 873 814
- US-A1- 2002 151 848
- US-A1- 2007 239 172
- US-A1- 2010 200 002
- US-A1- 2010 292 707
- US-A1- 2014 338 676
- US-A1- 2015 090 063

## Description

### CROSS REFERENCE TO RELATED APPLICATIONS

This application claims priority benefit of U.S. Provisional Application Serial Number 62/196,073 filed 23 July 2015.

### FIELD OF THE INVENTION

The present invention generally relates to robotic systems, and more specifically to a protective drape for robotic systems.

### BACKGROUND OF THE INVENTION

Robotic systems have been developed to aid in a variety of different applications ranging from the industrial, medical, and military fields. Robotic systems have unique characteristics to perform different tasks depending on the application to be performed, where the size, weight, geometry, construction, controls, programs and functionality are all characteristics considered when designing a robot.

Robotic systems are required to operate in varied environments that range from industrial applications to a sterile surgical suite for patient care or a clean room for manufacturing sensitive electronic components. In each of these applications there is a need to prevent contaminants from infiltrating the robot and affecting operation, as well to prevent contaminants from the robot infecting a patient or contaminating product being assembled or tested by the robot. Furthermore, in surgical or medical environments sterile conditions are required to be maintained to prevent the transfer of infectious agents between successive patients being treated by a robot.

A robotic system used in surgery must either be sterile or covered by a sterile drape, while an industrial robot often has a similar protective cover. Often, part of the robotic system must extend beyond the drape so that those parts can interact with the environment beyond the drape to perform desired tasks. In a robotic system with a large range of motion, fixing a drape to the operational end of the robotic system often leads to drape constriction. The resulting twisting and tightening of the drape around the robotic system requires manual adjustments of the drape. This may be alleviated by using a protective drape that is larger than needed to cover the system. With a larger protective drape the robot manipulator has more room to articulate reducing the chances of drape constriction. However, this excess material can interfere with the surgical procedure and use of the device. In addition, a larger protective drape requires additional drape material that is counterintuitive to install since the drape may not resemble the shape of the robot. On the other hand, a protective drape that has a conforming shape to the robot can be difficult to maneuver around the robot during installation.

Currently, tubes that are used to deliver fluid, such as sterile irrigation fluid, to the end effector of the robot must be routed around a protective drape after the drape is installed. This can be a difficult process, particularly keeping the irrigation tube sterile during installation and having the tube oriented in the correct direction. Additionally, exchange of such tubes is often required between surgical procedures owing to the external placement.

Thus there is a need in the art for protective draping for robotic systems that are easy to install, does not restrict the robots movement, and allows the robotic system to perform a specified task.

US 2002/151848 A1 discloses a protective cover for an elongated instrument comprising a hollow elongated member and at least one loop member. The elongated member has a first and second end at opposite ends thereof. At least a portion of the elongated member is a pliable material. A loop member extends along a generally circumferential direction or around a perimeter of the elongated member at the first end. The loop member is affixed to and supports the elongated member along at least a portion of a circumference or perimeter of the elongated member such that an opening is formed at the first end of the elongated member, and such that when in a relaxed configuration, the opening remains open without a force being applied to the loop member. The proportions of the height and longitudinal dimensions of the loop member cross-section are such that the loop member has enough structural strength the maintain the opening at the first end, even in a relaxed state.

### SUMMARY OF THE INVENTION

The invention is defined in claims 1 and 13. Preferred embodiments are defined in the dependent claims.

A protective drape for a robotic system is provided that includes a protective tube encapsulating at least a portion of the robotic system with a first ring connected to, or in proximity to a first end perimeter of the protective tube and adapted to fit around a distal portion of the robotic system. A second ring is connected to a second end perimeter of the protective tube, where the second ring is configured to fit around a proximal portion of the robotic system without constraining the rotation of a robotic arm. One or more draw cords are provided to remove excess slack at different points along the length of the protective tube and the robotic arm.

The rings are rotationally unconstrained.

A protective drape for a robotic system is provided that includes a plurality of drape segments adapted to collectively encapsulate at least a portion of the robotic system, with one or more sectional rings positioned between and joining the plurality of drape segments. A first ring is connected to a first end perimeter of the collective drape segments, where the first ring is configured to fit around a distal portion of the robotic system, and a second ring is connected to a second end perimeter of the collective drape segments, where the second ring is configured to fit around a proximal portion of the robotic system.

A protective drape for a robotic system is provided that includes a protective tube having a length configured to encapsulate a robotic arm. A first ring is connected to a first end perimeter of the protective tube, where the first ring is configured to fit around a distal portion of the robotic arm, and a second ring is connected to a second end perimeter of the protective tube, where the second ring is configured to fit around a proximal portion of the robotic arm, and an irrigation, vacuum, or air tube/line is integrated with the protective tube.

A method is provided for deploying a protective drape to encapsulate a robotic system, where the method includes holding a tube of the protective drape connected to at least one of a pair of rings in front of the robotic system; and automatically moving or advancing a robotic arm of the robotic system through the protective tube to encapsulate the robotic system, wherein said rings are rotationally unconstrained.

### BRIEF DESCRIPTION OF THE DRAWINGS

The present invention is further detailed with respect to the following drawings that are intended to show certain aspects of the present invention, but should not be construed as a limit on the practice of the present invention.
FIG. 1 is a perspective view of a robotic system with a transparent protective drape joined to a robotic arm with rotationally unconstrained rings at the fixed base of the robot and at the rotating tool attachment of an end effector in accordance with the invention;
FIG. 2 is a perspective view of a robotic system with a transparent protective drape joined to a robotic arm that includes a blower to control a desired pressure within the protective drape in accordance with embodiments of the invention;
FIG. 3 is a perspective view of a protective drape in a folded state to aid in transportation and to facilitate installation in accordance with embodiments of the invention;
FIG. 4 is a perspective view of a robotic system with a segmented transparent protective drape joined with sectional rings, and joined to a robotic arm with rotationally unconstrained rings at the fixed base of the robot and at the rotating tool attachment point of an end effector in accordance with embodiments of the invention;
FIG. 5 is a perspective view of a robotic system with a segmented transparent protective drape joined with sectional rings, where the sectional rings are joined to the robot arm, and the ends of the protective drape are joined to a robotic arm with rotationally unconstrained rings at the fixed base of the robot and at the rotating tool attachment point of an end effector in accordance with embodiments of the invention;
FIG. 6 is a perspective view of a robotic system with a transparent protective drape with draw cords to remove excess slack at different points along the length of the protective drape, the protective drape joined to a robotic arm with rotationally unconstrained rings at the fixed base of the robot and at the rotating tool attachment point of an end effector in accordance with embodiments of the invention;
FIG. 7 is a perspective view of a robotic system with a transparent protective drape with an irrigation, vacuum, or air tube/line forming a skeleton along the length of the protective drape, the protective drape joined to a robotic arm with rotationally unconstrained rings at the fixed base of the robot and at the rotating tool attachment point of an end effector in accordance with embodiments of the invention; and
FIG. 8 is an illustrative depiction of a protective drape with two protective tubes to control fluid flow for temperature control in accordance with embodiments of the invention.

### DETAILED DESCRIPTION OF THE INVENTION

The present invention has utility as a protective draping for a robot of a robotic system. The following description of various embodiments of the invention is not intended to limit the invention to these specific embodiments, but rather to enable any person skilled in the art to make and use this invention through exemplary aspects thereof.

It is to be understood that in instances where a range of values are provided that the range is intended to encompass not only the end point values of the range but also intermediate values of the range as explicitly being included within the range and varying by the last significant figure of the range. By way of example, a recited range from 1 to 4 is intended to include 1-2, 1-3, 2-4, 3-4, and 1-4.

Embodiments of the present invention describe a protective drape for, and method of usage with a robotic system. It should be appreciated that any autonomous (i.e., active), semi-autonomous, passive, or haptic robotic system either for medical or industrial applications may benefit from the system and methods disclosed herein. Embodiments of the invention may be used with robotic systems that are required to operate in varied environments that illustratively include industrial applications, a sterile surgical suite for patient care, and a clean room for manufacturing sensitive electronic components. In each of these applications where embodiments of the invention are employed, there is a need to prevent contaminants from infiltrating from the environment to the robot and affecting operation of the robot itself or the robotic system, as well to prevent contaminants from the robot from infecting a patient or contaminating an assembly or process product. Furthermore, in surgical or medical environments sterile conditions are required to be maintained to prevent the transfer of infectious agents between patients being treated by the robots; the present invention addresses these requirements with the deployment of a protective drape that is different and superior to the prior art.

Embodiments described herein make reference to a robotic system. It should be understood that the robotic system may further include external components such as external hardware and software, tracking systems, external user interfaces and external user input mechanisms. The external components may require additional protective draping independent of the robotic system described herein, unless otherwise stated. Examples of the components and control of a robotic system are described in U.S. Pat. App. 12/703,125 and U.S. Pat. 5,806,518. Examples of such robotic systems include the LBR iiwa Lightweight Industrial Robot Series (KUKA Robotics Corp., Shelby Township, Michigan), the ROBODOC® Surgical System (THINK Surgical, Inc., Fremont, CA), and the RIO® Robotic System (Mako Surgical Corp., Ft. Lauderdale, FL).

Embodiments of the protective drape may be made of paper, woven materials, fabrics, plastic films, plastic sheets, foils, and combinations thereof. In certain inventive embodiments, an inventive drape is formed with transparent plastic film to allow visual inspection of an encapsulated robot without compromising the position or integrity of the protective drape.

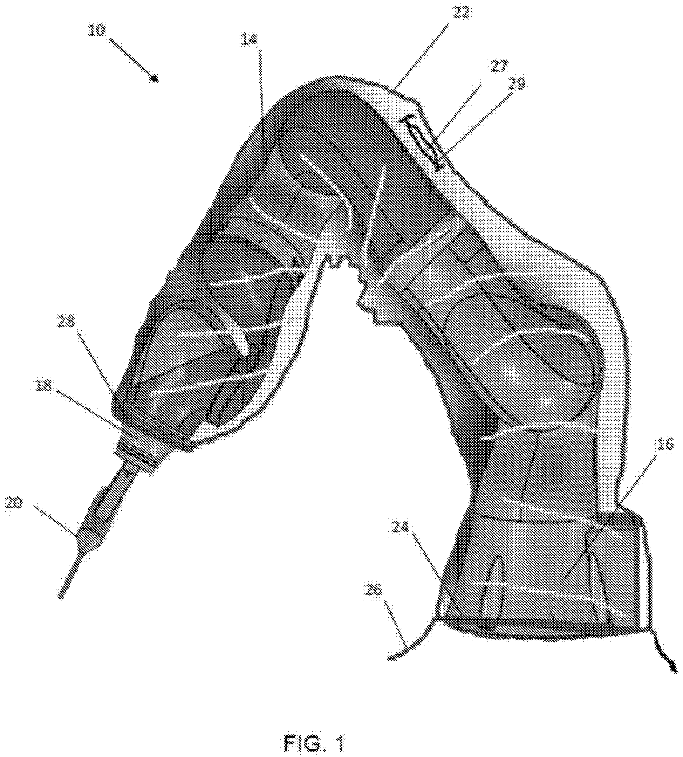

Referring now to the figures, FIG. 1 depicts a draped robotic system 10 in accordance with the invention. The robotic system 10 may include a segmented robotic arm 14 that rotates on a base 16, and an end effector manipulator 18 that rotates relative to the robotic arm 14. A most distal portion of the robotic system 10 is an end effector 20 that may be removably attached to the end effector manipulator 18. The most proximal portion of the robotic system 10 is the base 16. The segments of the robotic arm 14 may be connected by various revolute, prismatic or spherical joints to actuate the end effector 20 in one or more degrees of freedom, preferably five or more. The protective drape 22 is formed of a sheet material formed as a tube that is joined to the robotic system 10 with rotationally unconstrained rings 24 and 28. The ring 28 may be joined to a distal portion of the robotic system 10, such as the end effector 20, or the end effector manipulator 18. Ring 24 may be joined to a more proximal portion of the draped robotic system 10 such as the base 16 or a proximal segment of the robotic arm 14. For the purposes of visual clarity, the drape 22 is depicted as being formed of a transparent material, such as polyethylene; however, it is appreciated that paper, woven materials, fabrics, non-transparent plastic films or sheets, foils, and combinations thereof are also suitable for the formation of the drape 22. The protective tube that forms the protective drape 22 may be formed according to the shape and size of the robotic system 10. For example, the protective tube may be formed or manufactured in the shape of a hollow cylinder, hollow square, or of any hollow irregular shape depending on the type or geometry of the robotic system 10. Additionally, the protective tube may be readily formed by joining opposing sheet edges to define, for example a hollow cylinder, or may be formed by a seamless film or foil, by conventional techniques. It is also appreciated that luminal segments of like or different material are readily joined to form a segmented protective drape. Alternatively, swatches of sheet material may be joined to form a tube with a quilt-like pattern.

In some examples the ring 28 engages the end effector 20. The use of rotationally unconstrained rings (24, 28) allows the drape 22 to be attached to the end effector 20 directly rather than a link proximal to the last revolute joint, while the end effector 20 is free to rotate an unlimited number of rotations without constricting the drape 22, and allows the wrist to perpetually invert while holding the end effector position for an unlimited number of rotations. The use of rings reduces the size of the drape for easy installation.

In some inventive embodiments, the drape 22 has an opening 27 in a preselected position to provide access to the arm 14 or base 16. A seal 29 provides for closure of the opening 27. The seal 29 includes a contact adhesive strip, or a fastening structure as detailed in U.S. Pat. 5,809,621. Typically the drape is a continuous sheet with adhesives to combine multiple drape components. However, having an opening with a seal can be advantageous if there are controls on the arm or on the base that require access. It is appreciated that a seal 29 is readily formed such that a sterile barrier exists between the robot 12 and the exterior environment.

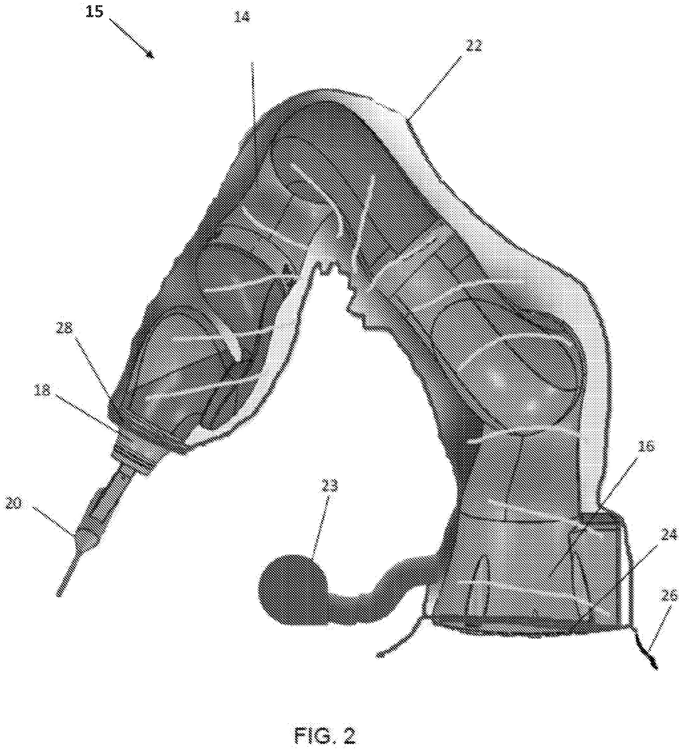

In still other embodiments of the present invention, with respect to FIG. 2, a robotic system 15 includes a blower, vacuum, or air compressor 23 to control the pressure within the protective drape 22. Depending on the nature of the work environment, the draped robotic system 10 has either a positive pressure or negative pressure within the protective drape 22 relative to the surrounding environment. By way of example, in an industrial setting with corrosive or particle ladened environment, a blower 23 may be used to create positive pressure inside the drape 22, protecting the robotic system 15 and further serves to push the sheet material of the drape 22 away from moving components of the robotic system 15. In a medical setting, it may be advantageous to have a vacuum 23 to create negative pressure within the protective drape 22 such that particles or debris on the robotic system 15 cannot escape or contaminate the surgical field. In certain inventive embodiments, the robotic system 15 may include a pressure control system to control the direction of airflow. Therefore, depending on the application or stage of a procedure, the area between the robotic system 15 and the protective drape 22 may be toggled between a positive or negative pressure state. Additionally, one or more of the rings (24, 28) may provide a hermetic seal to help control the pressure within the drape 22. Although, a hermetic seal may not be necessary if the pressure differential is sufficient to maintain the pressure state.

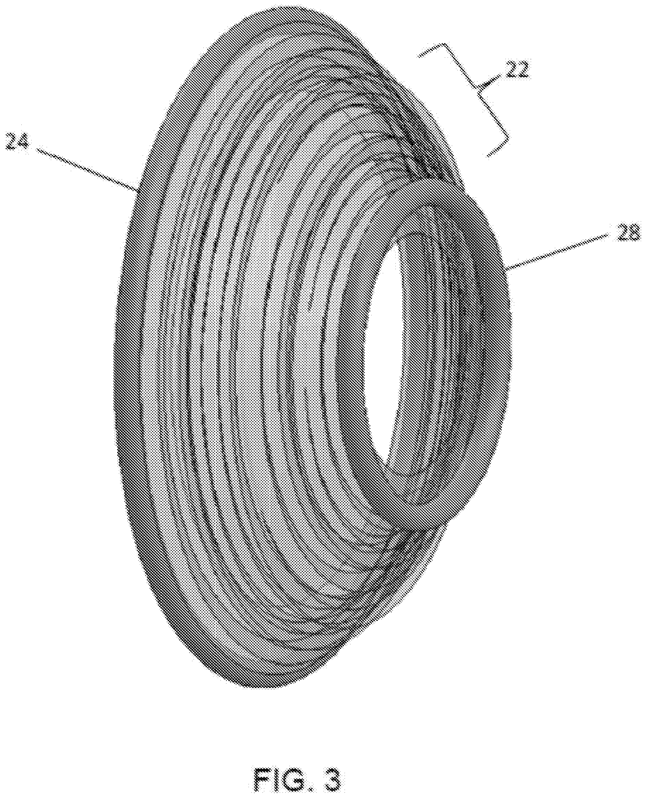

In a particular inventive embodiment, with respect to FIGs. 1 and 3, a set of rings (24, 28), where one ring 28 is approximately the size of the end effector manipulator 18 or end effector 20, or a ring that does not constrain rotation (i.e., a "slip ring") of the end effector manipulator 18 or end effector 20 is used at the distal end of the protective drape 22. The second ring 24 is a simple ring that is larger than the largest diameter of the robot arm 14 and base 16. The protective drape 22 may be compressed or folded between the two rings (24, 28) as shown in FIG. 3. For example, the protective drape 22 may have an accordion-type fold similar to that of a bellow. When deployed, as shown in FIG. 1, the drape 22 may be shaped like the robotic arm 14 in a manner that the robotic system 10 is able to be moved throughout its entire range of motion without cinching the drape 22. It is appreciated that cinching of the sheet material of the drape 22 can limit the operational range of the robotic system 10 in general, and the end effector 20 in particular; additionally, the barrier function of the drape 22 is compromised if the cinching results in a tear.

In a particular inventive method, the protective drape 22 is installed with a user holding the rings (24, 28) in front of the robotic system 10. The first ring 28 of the drape 22 is fixed to the distal end of the robotic system 10 (end effector 20 if attached, or end effector manipulator 18 if the end effector 20 is not attached) by a user. The robotic arm 14 then automatically moves or advances through the rings (24, 28) in a manner that the user does not need to move the second ring 24. As the robotic arm 14 moves through the second ring 24, the drape 22 unfolds until the user is holding the second ring 24 near the base 16 of the robotic system 10. In a specific embodiment, the user manually unfolds the drape 22 by holding the first ring 28 and moving the second ring 24 down the length of the arm 14. In another embodiment, a separate device (not shown) holds the two rings (24, 28) of the folded drape 22. The robotic system 10 automatically moves the end effector 20 to the device, where the end effector 20 either automatically attaches to the first ring 28, or the user manually attaches the first ring 28 to the end effector. Subsequently, the robotic system 10 either moves the arm 14 through the second ring 24, while the second ring 24 remains stationary, or, the robotic system 10 "picks up" the drape 22, lifts the drape 22 above the arm 14, and allows gravity to unfold the drape 22 along the robotic arm 14. After the second ring 24 is at the base 16 of the robotic system 10, the second ring 24 may be attached to a base drape 26 to create a base seal. The base drape 26 may drape any additional components beyond the base 16 of the robotic system 10, such as a supporting structure supported on the floor or a wall that might contain additional robotic hardware components.

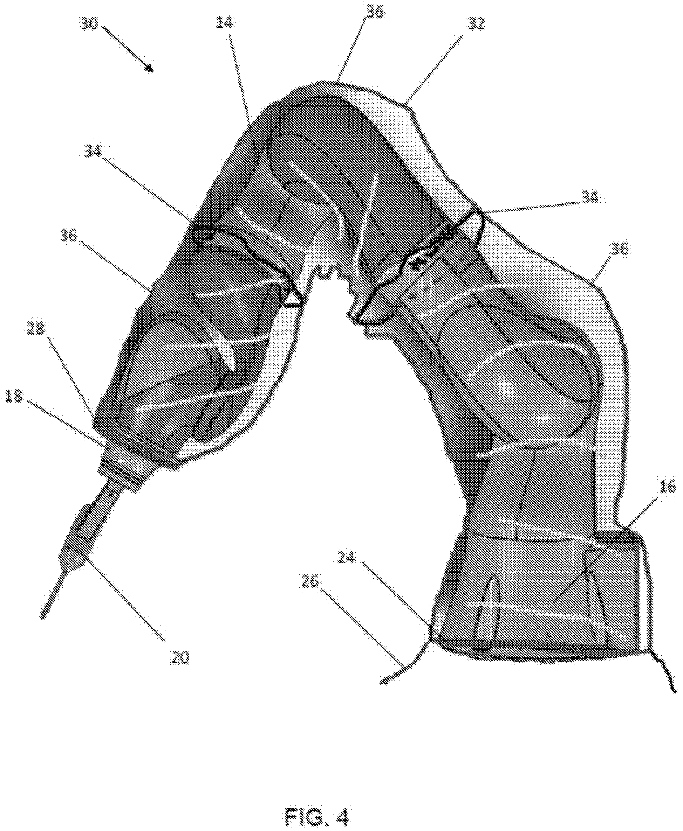

FIG. 4 is a perspective view of a robotic system 30 with a segmented protective drape 32 having drape segments 36 that are joined by sectional rings 34 in accordance with embodiments of the invention. The segmented protective drape 32 is joined to a robotic arm 14 with rotationally unconstrained rings (24, 28) at the fixed base 16 of the robotic system 30, and at the end effector manipulator 18 or end effector 20. With respect to FIG. 4, like numerals have the meaning ascribed thereto with respect to the preceding drawing. In a specific embodiment, the sectional rings 34 may form a rigid connection between the segments 36, or the sectional rings 34 may form a rotationally-unconstrained connection between the segments 36.

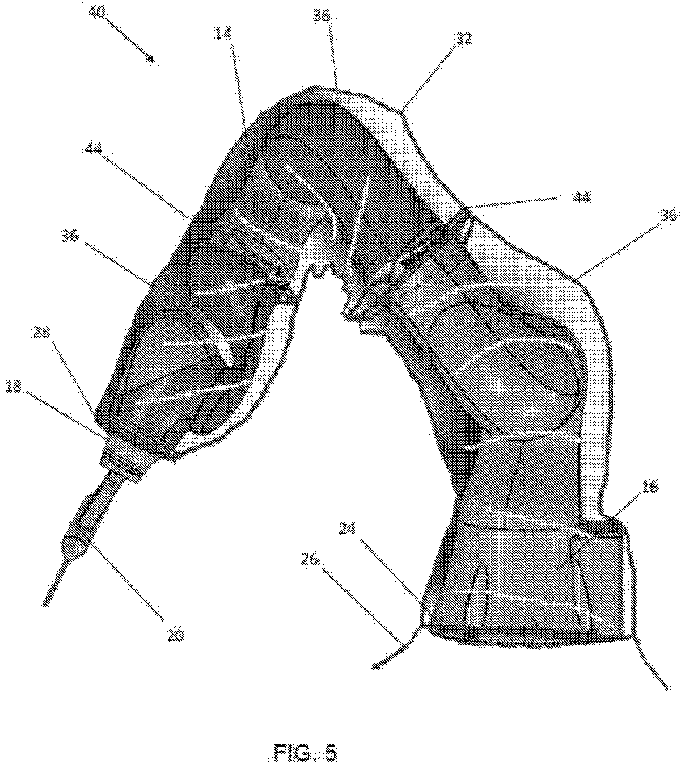

FIG. 5 is a perspective view of a robotic system 40 with a segmented protective drape 32 joined with sectional rings 44, where the sectional rings 44 are joined or clipped onto the robot arm 14 in accordance with embodiments of the invention. The ends of the protective drape 32 are joined to a robotic arm 14 with rotationally unconstrained rings (24, 28) at the fixed base 16 of the robotic system 40, and at the end effector manipulator 18 or end effector 20. With respect to FIG. 5, like numerals have the meaning ascribed thereto with respect to the preceding drawings.

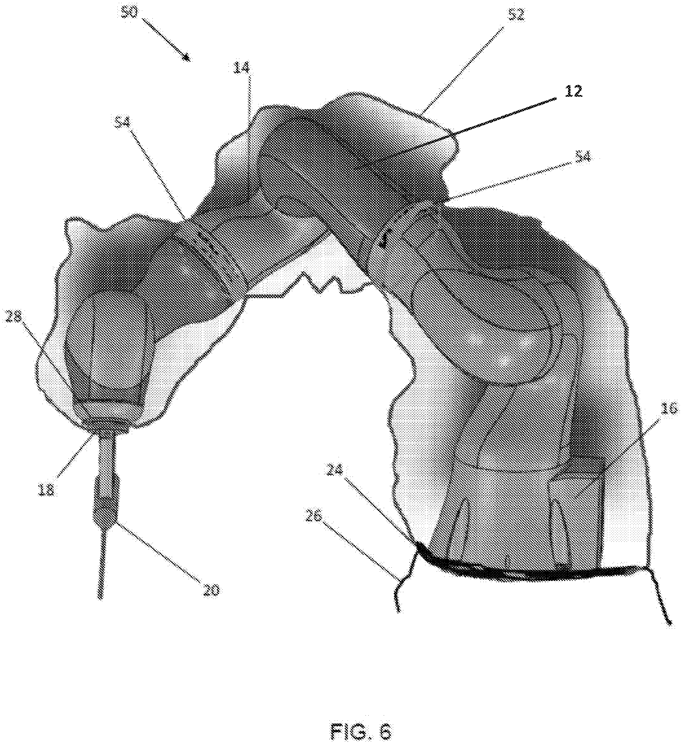

FIG. 6 is a perspective view of an embodiment of a robotic system 50 with a protective drape 52 with draw cords 54 to remove excess slack at different points along the length of the protective drape 52. With respect to FIG. 6, like numerals have the meaning ascribed thereto with respect to the preceding drawings. The protective drape 52 is joined to a robotic arm 14 with rotationally unconstrained rings at the fixed base 16 of the robot 12 and at the end effector manipulator 18 or end effector 20. The use of draw cords 54 allows for a uniform-sized drape with uniform cross-section which is easier to manufacture, while allowing slack where needed but preventing excess slack where it is not needed. In a specific embodiment, a tube-shaped drape 52 that has a larger diameter as the largest diameter section of the robotic system 50, and allows the necessary slack to prevent twisting during rotation of the arm 14, has locations along the length of the drape 52 where the cross-section of the robotic arm 14 is smaller and excess slack is not needed, draw cords 54 are placed within the drape 52 to allow the drape 52 to be cinched against the robot 12.

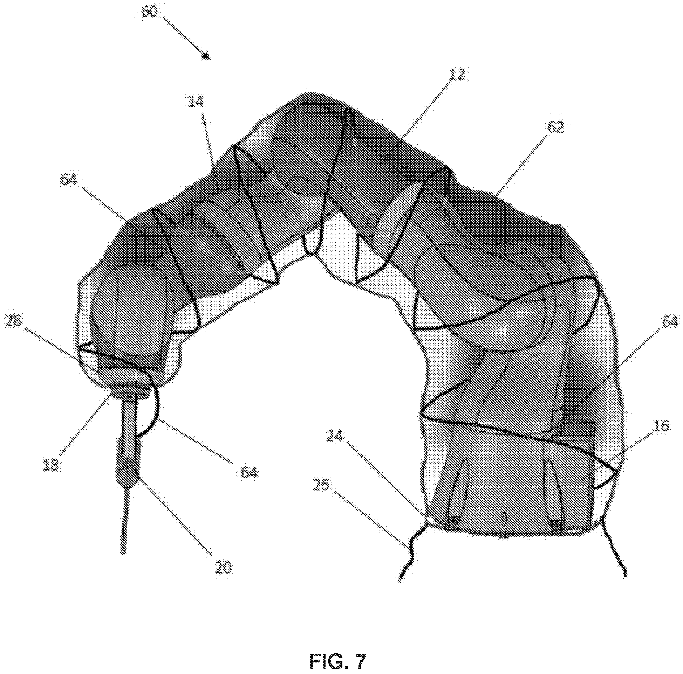

FIG. 7 is a perspective view of a robotic system 60 with a protective drape 62 with an irrigation, vacuum, or air tube/line 64 forming a skeleton along the length of the protective drape 62 in accordance with embodiments of the invention. The protective drape 62 is joined to the robotic arm 12 with rotationally unconstrained rings (24, 28) at the fixed base 16 of the robotic system 60 and at the end effector manipulator 18 or end effector 20. With respect to FIG. 7, like numerals have the meaning ascribed thereto with respect to the preceding drawings. In a specific embodiment the irrigation, vacuum, or air tube/line 64 is in a helical or spring like pattern along the length of the protective drape 62.

In a specific embodiment, the irrigation, vacuum, or air tube/line 64 may come pre-packaged and folded with the drape 62 such that unfolding the drape 62 also unwinds the irrigation tube/line 64 such that the tube 62 is in an optimal position on the robot 12 and ensures that the correct end of the irrigation tube 62 is at the end effector 18 when the drape 62 is deployed. In specific embodiments, the irrigation tube 62 may also provide an external "skeleton" to the drape 62, preventing the drape 62 from binding up and facilitating an easier installation of the drape 62. The irrigation tube/line 64 could be coiled such that unfolding the drape 62 keeps the drape 62 coiled around the robot 12.

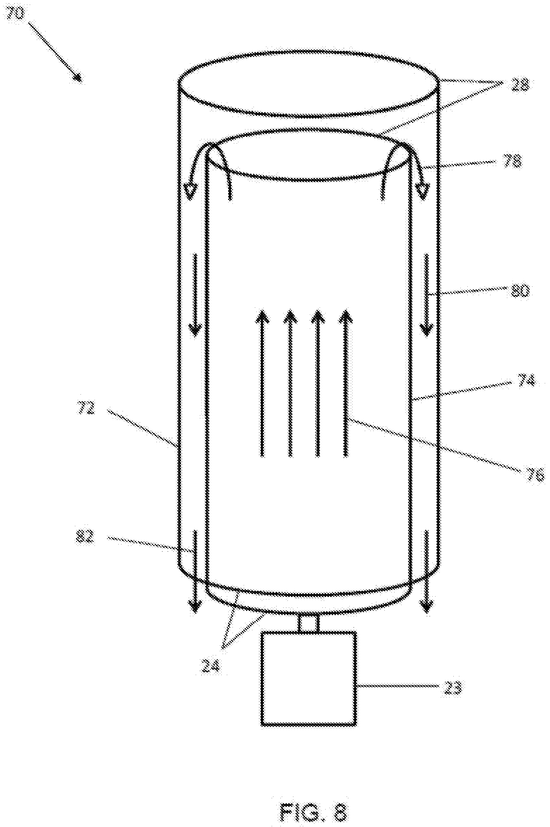

In a particular inventive embodiment, with respect to FIG. 8, a protective drape 70 for a robotic system may include an outer tube 72 and a second inner tube 74 incorporated within, wherein a fluid (e.g., cooling air) can pass there between. A blower 23 may be attached to the inner tube 74 near ring 24 to blow fluid over the components of the robotic system (i.e., direction of arrows 76) encapsulated within the inner tube 74 of the protective drape 70. The fluid may then come out inside of the protective drape 70 near the front of the inner tube 74 and then through the space between the outer tube 72 and inner tube 74 (fluid flow direction is indicated by arrows 78 and 80). The fluid may then exit (indicated by arrow 82) back out near the blower 23 between the inner tube 74 and outer tube 72. The protective drape 70 will keep the temperature of the robotic system within the inner tube 74 down, which otherwise could increase due to the enclosing of the robot hardware.

The rings 24 and 28 of protective drape 70 may be attached to both the inner tube 74 and the outer tube 72. In a specific embodiment, ring 28 is attached to both the inner tube 74 and the outer tube 72, while ring 24 is only attached to the inner tube 74 to allow all the fluid to exit near the blower. If ring 28 is attached to both the inner tube 74 and the outer tube 72, holes or other perforations may exist near the distal portion of the inner tube to allow fluid to pass to the space between the inner tube 74 and outer tube 72. Therefore, the fluid may not expel near the end effector 20 where the surgical procedure is performed.

## Claims

1. A protective drape (22, 32, 52, 62, 70) for a robotic system (10, 15, 30, 40, 50, 60) comprising:
a protective tube or a plurality of drape segments (36) encapsulating at least a portion of said robotic system (10, 15, 30, 40, 50, 60);
a first ring (28) connected to, or in proximity to a first end perimeter of said protective tube or said collective drape segments (36) and adapted to fit around a distal portion of said robotic system (10, 15, 30, 40, 50, 60);
a second ring (24) connected to a second end perimeter of said protective tube or said collective drape segments (36), wherein said second ring (24) is configured to fit around a proximal portion of said robotic system (10, 15, 30, 40, 50, 60) without constraining the rotation of a robotic arm (14);
and one or more draw cords (54) to remove excess slack at different points along the length of said tube and said robotic arm (14),
**characterized in that**
said rings (24, 28) are rotationally unconstrained.

2. The protective drape (22, 32, 52, 62, 70) of claim 1,
wherein said protective drape (22, 32, 52, 62, 70) is either made of paper, woven materials, fabrics, plastic films, plastic sheets, foils, or a combination thereof;
or is a plastic film that is transparent;
or said protective drape (22, 32, 52, 62, 70) is folded between said first ring (28) and said second ring (24) prior to encapsulating said robotic system (10, 15, 30, 40, 50, 60).

3. The protective drape (22, 32, 52, 62, 70) of any one of claims 1 or 2, which is adapted to be connected a blower (23) for intraluminal pressure control or to at least one of a blower (23), a vacuum, or air compressor, to control the pressure between said robotic system (10, 15, 30, 40, 50, 60) and said protective drape (22, 32, 52, 62, 70).

4. The protective drape (22, 32, 52, 62, 70) of any one of claims 1 or 2, comprising an access opening (27) in a wall of said protective tube and with a closure seal (29) adapted to selectively seal said access opening (27).

5. The protective drape (22, 32, 52, 62, 70) of any one of claims 1 or 2,
wherein said second ring forms (24) a hermetic seal with said robotic system (10, 15, 30, 40, 50, 60).

6. The protective drape (22, 32, 52, 62, 70) of claim 1,
wherein said first ring (28) is adapted to fit around an end effector (20) of said robotic system (10, 15, 30, 40, 50, 60) and said second ring (24) is configured to fit around a base portion (16) of said robotic system (10, 15, 30, 40, 50, 60) without constraining the rotation of said robotic arm (14).

7. The protective drape (22, 32, 52, 62, 70) of claim 6,
wherein said first ring (28) fits around said end effector (20) of the robotic system (10, 15, 30, 40, 50, 60) without constraining the rotation of the end effector (20), and
wherein said first ring (28) preferably forms a hermetic seal to said robotic system (10, 15, 30, 40, 50, 60).

8. The protective drape (22, 32, 52, 62, 70) of claim 1,
wherein said one or more sectional rings (34) form either a rigid or a rotationally un-constrained connection between said plurality of drape segments (36), or said one or more sectional rings (34) are connected to the robotic arm (14).

9. The protective drape (22, 32, 52, 62, 70) of any one of claims 1 or 8,
wherein each of said drape segments (36) are independently either made of paper, woven materials, fabrics, plastic films, foils, or combinations thereof, or are plastic films that are transparent.

10. The protective drape (22, 32, 52, 62, 70) according to one of claims 1 to 7,
wherein the protective tube has a length configured to encapsulate the robotic arm (14); and
an irrigation, vacuum, or air tube/line (64) integrated with said protective tube.

11. The protective drape (22, 32, 52, 62, 70) of claim 10,
wherein said irrigation, vacuum, or air tube/line (64) is in a helical or spring like pattern along the length of said protective tube and preferably either forms a skeleton along the length of said protective tube or is pre-packaged and folded with the drape (22, 32, 52, 62, 70), where unfolding the drape (22, 32, 52, 62, 70) also unwinds said irrigation, vacuum, or air tube/line (64); and
wherein an end of said irrigation, vacuum, or air tube/line (64) is positioned at the end effector (20) when said protective tube is deployed.

12. A robotic system (10, 15, 30, 40, 50, 60) comprising:
a base (16);
a robotic arm (14) having at least three degrees of freedom extending from the base (16); and a protective drape (22, 32, 52, 62, 70) of any one of claims 1 to 11.

13. A method of deploying a protective drape (22, 32, 52, 62, 70) to encapsulate a robotic system (10, 15, 30, 40, 50, 60) comprising:
holding a tube of the protective drape (22, 32, 52, 62, 70) connected to at least one of a pair of rings (24, 28) in front of said robotic system(10, 15, 30, 40, 50, 60); and
automatically moving or advancing a robotic arm (14) of said robotic system (10, 15, 30, 40, 50, 60) through the protective tube to encapsulate said robotic system (10, 15, 30, 40, 50, 60),
**characterized in that**
said rings (24, 28) are rotationally unconstrained.

14. The method of claim 13,
wherein said protective drape (22, 32, 52, 62, 70) unfolds as said robotic arm (14) moves through a second ring (24) of said pair of rings (24, 28), preferably held by a human user and/or a device.

15. The method of claim 14,
wherein the protective tube is connected to a first ring (28) of said pair of rings (24, 28), the first ring (28) being affixed to a distal end of the robotic system (10, 15, 30, 40, 50, 60),
wherein said pair of rings (24, 28) are affixed to said protective tube after the tube surrounds the robotic system (10, 15, 30, 40, 50, 60),
and/or the method comprising creating a base seal by attaching said second ring (24) of said pair of rings (24, 28) to a base (16) of the robotic system (10, 15, 30, 40, 50, 60).

## Patentansprüche

1. Schutzabdeckung (22, 32, 52, 62, 70) für ein Robotersystem (10, 15, 30, 40, 50, 60), umfassend:
einen Schutzschlauch oder eine Vielzahl von Abdecksegmenten (36), die mindestens einen Teil des Robotersystems (10, 15, 30, 40, 50, 60) einkapseln;
einen ersten Ring (28), der mit einem ersten Endumfang des Schutzschlauchs oder der kollektiven Abdecksegmenten (36) verbunden ist oder
sich in der Nähe davon befindet und so angepasst ist, dass er um einen distalen Teil des Robotersystems (10, 15, 30, 40, 50, 60) passt;
einen zweiten Ring (24), der mit einem zweiten Endumfang des Schutzrohrs oder der kollektiven Abdecksegmente (36) verbunden ist, wobei der zweite Ring (24) so geformt ist, dass er um einen proximalen Abschnitt des Robotersystems (10, 15, 30, 40, 50, 60) passt, ohne die Drehung eines Roboterarms (14) einzuschränken;
und eine oder mehrere Zugschnüre (54), um überschüssige Spiel an verschiedenen Punkten entlang der Länge des Rohrs und des Roboterarms (14) zu entfernen,
**dadurch gekennzeichnet, dass**
die Ringe (24, 28) in ihrer Drehung nicht eingeschränkt sind.

2. Schutzabdeckung (22, 32, 52, 62, 70) nach Anspruch 1,
wobei die Schutzabdeckung (22, 32, 52, 62, 70) entweder aus Papier, gewebte Materialien, Geweben, Plastikfilmen, Plastikfolien, Folien oder einer Kombination davon hergestellt ist;
oder ein durchsichtiger Plastikfilm ist;
oder die Schutzabdeckung (22, 32, 52, 62, 70) wird zwischen dem ersten Ring (28) und dem zweiten Ring (24) gefaltet, bevor das Robotersystem (10, 15, 30, 40, 50, 60) eingekapselt wird.

3. Schutzabdeckung (22, 32, 52, 62, 70) nach einem der Ansprüche 1 oder 2, der geeignet ist, ein Gebläse (23) zur intraluminalen Drucksteuerung oder mit mindestens einem von einem Gebläse (23), einem Vakuum oder Luftkompressor verbunden zu werden, um den Druck zwischen dem Robotersystem (10, 15, 30, 40, 50, 60) und der Schutzabdeckung (22, 32, 52, 62, 70) zu steuern.

4. Schutzabdeckung (22, 32, 52, 62, 70) nach einem der Ansprüche 1 oder 2, umfassend einer Zugangsöffnung (27) in einer Wand des Schutzschlauches und mit einer Verschlussdichtung (29), die geeignet ist, die Zugangsöffnung (27) selektiv abzudichten.

5. Schutzabdeckung (22, 32, 52, 62, 70) nach einem der Ansprüche 1 oder 2, wobei der zweite Ring (24) eine hermetische Dichtung mit dem Robotersystem (10, 15, 30, 40, 50, 60) bildet.

6. Schutzabdeckung (22, 32, 52, 62, 70) nach Anspruch 1,
wobei der erste Ring (28) so angepasst ist, dass er um einen Endeffektor (20) des Robotersystems (10, 15, 30, 40, 50, 60) passt und der zweite Ring (24) so konfiguriert ist, dass er um einen Basisabschnitt (16) des Robotersystems (10, 15, 30, 40, 50, 60) passt, ohne die Drehung des Roboterarms (14) einzuschränken.

7. Schutzabdeckung (22, 32, 52, 62, 70) nach Anspruch 6,
wobei der erste Ring (28) um den Endeffektor (20) des Robotersystems (10, 15, 30, 40, 50, 60) passt, ohne die Drehung des Endeffektors (20) einzuschränken, und
wobei der erste Ring (28) vorzugsweise eine hermetische Abdichtung zu dem Robotersystem (10, 15, 30, 40, 50, 60) bildet.

8. Schutzabdeckung (22, 32, 52, 62, 70) nach Anspruch 1,
wobei der eine oder die mehreren Teilringe (34) entweder eine starre oder eine drehfreie Verbindung zwischen der Vielzahl von Abdecksegmenten (36) bilden oder der eine oder die mehreren Teilringe (34) mit dem Roboterarm (14) verbunden sind.

9. Schutzabdeckung (22, 32, 52, 62, 70) nach einem der Ansprüche 1 oder 8, wobei jedes der Abdecksegmente (36) unabhängig entweder aus Papier, gewebten Materialien, Geweben, Stoffen, Plastikfilmen, Folien oder Kombinationen davon hergestellt sind oder transparente Plastikfilme sind.

10. Schutzabdeckung (22, 32, 52, 62, 70) nach einem der Ansprüche 1 bis 7, wobei der Schutzschlauch eine Länge hat, die so konfiguriert ist, dass sie den Roboterarm (14) einkapselt; und
einen/eine Spül-, Vakuum- oder Luftschlauch/-leitung (64), der/die in den Schutzschlauch integriert ist.

11. Schutzabdeckung (22, 32, 52, 62, 70) nach Anspruch 10,
wobei der/die Spül-, Vakuum- oder Luftschlauch/-leitung (64) in einem spiralförmigen oder federartigen Muster entlang der Länge des Schutzschlauches ist und vorzugsweise entweder ein Skelett entlang der Länge des Schutzschlauches bildet oder vorverpackt und mit der Abdeckung (22, 32, 52, 62, 70) gefaltet ist, wobei das Entfalten der Abdeckung (22, 32, 52, 62, 70) auch den/die Spül-, Vakuum- oder Luftschlauch/-leitung (64) abwickelt; und wobei ein Ende des/der Spül-, Vakuum- oder Luftschlauch/-leitung (64) am Endeffektor (20) positioniert ist, wenn der Schutzschlauch entfaltet ist.

12. Robotersystem (10, 15, 30, 40, 50, 60), umfassend:
eine Basis (16);
einen Roboterarm (14) mit mindestens drei Freiheitsgraden, der sich von der Basis (16) aus erstreckt; und eine Schutzabdeckung (22, 32, 52, 62, 70) nach einem der Ansprüche 1 bis 11.

13. Verfahren zum Entfalten einer Schutzabdeckung (22, 32, 52, 62, 70) zum Einkapseln eines Robotersystems (10, 15, 30, 40, 50, 60), umfassend:
Halten eines Schlauchs der Schutzabdeckung (22, 32, 52, 62, 70) verbunden mit mindestens einem eines Paars von Ringen (24, 28) vor dem Robotersystem (10, 15, 30, 40, 50, 60); und
automatisches Bewegen oder Vorschieben eines Roboterarms (14) des Robotersystems (10, 15, 30, 40, 50, 60) durch den Schutzschlauch, um das Robotersystem (10, 15, 30, 40, 50, 60) einzukapseln,
**dadurch gekennzeichnet, dass**
besagte Ringe (24, 28) rotationsfrei sind.

14. Verfahren nach Anspruch 13,
wobei sich die Schutzabdeckung (22, 32, 52, 62, 70) entfaltet, wenn sich der Roboterarm (14) durch einen zweiten Ring (24) des Paars von Ringen (24, 28) bewegt, vorzugsweise von einem menschlichen Benutzer und/oder einer Vorrichtung gehalten.

15. Verfahren nach Anspruch 14,
wobei der Schutzschlauch mit einem ersten Ring (28) des Paars von Ringen (24, 28) verbunden wird, wobei der erste Ring (28) an einem distalen Ende des Robotersystems (10, 15, 30, 40, 50, 60) befestigt ist,
wobei das Paar von Ringen (24, 28) an dem Schutzschlauch befestigt werden, nachdem das Rohr das Robotersystem (10, 15, 30, 40, 50, 60) umgibt, und/oder wobei das Verfahren das Erzeugen einer Basisdichtung durch Befestigen des zweiten Rings (24) des Paars von Ringen (24, 28) an einer Basis (16) des Robotersystems (10, 15, 30, 40, 50, 60) umfasst.

## Revendications

1. Housse de protection (22, 32, 52, 62, 70) destinée à un système robotisé (10, 15, 30, 40, 50, 60) comprenant :
un tube de protection ou une pluralité de segments de housse (36) encapsulant au moins une portion dudit système robotisé (10, 15, 30, 40, 50, 60) ;
un premier anneau (28) connecté à ou à proximité d'un premier périmètre d'extrémité dudit tube de protection ou desdits segments de housse collectifs (36) et adapté pour s'ajuster autour d'une portion distale dudit système robotisé (10, 15, 30, 40, 50, 60) ;
un second anneau (24) connecté à un second périmètre d'extrémité dudit tube de protection ou desdits segments de housse collectifs (36), dans lequel ledit second anneau (24) est configuré pour s'ajuster autour d'une portion proximale dudit système robotisé (10, 15, 30, 40, 50, 60) sans contraindre la rotation d'un bras robotisé (14) ; et
un ou plusieurs cordons à tirer (54) pour éliminer un excédent de mou à différents points le long de la longueur dudit tube et dudit bras robotisé (14),
**caractérisé en ce que**
lesdits anneaux (24, 28) sont non contraints en rotation.

2. Housse de protection (22, 32, 52, 62, 70) selon la revendication 1,
dans laquelle ladite housse de protection (22, 32, 52, 62, 70) est soit réalisée en papier, en matériaux tissés, en tissus, en films de matière plastique, en panneaux de matière plastique, en feuilles ou une combinaison de ceux-ci ;
soit est un film de matière plastique et transparent ;
soit encore ledit housse de protection (22, 32, 52, 62, 70) est pliée entre ledit premier anneau (28) et ledit second anneau (24) avant d'encapsuler ledit système robotisé (10, 15, 30, 40, 50, 60).

3. Housse de protection (22, 32, 52, 62, 70) selon l'une quelconque des revendications 1 ou 2,
qui est adaptée pour être connecté à une soufflante (23) pour une commande de pression intraluminale ou à au moins un élément parmi une soufflante (23), une source de vide, ou un compresseur d'air pour commander la pression entre ledit système robotisé (10, 15, 30, 40, 50, 60) et ladite housse de protection (22, 32, 52, 62, 70).

4. Housse de protection (22, 32, 52, 62, 70) selon l'une quelconque des revendications 1 ou 2,
comprenant une ouverture d'accès (27) dans une paroi dudit tube de protection et comprenant un joint de fermeture (29) adapté pour étancher sélectivement ladite ouverture d'accès (27).

5. Housse de protection (22, 32, 52, 62, 70) selon l'une quelconque des revendications 1 ou 2,
dans laquelle ledit second anneau (24) forme un joint hermétique avec ledit système robotisé (10, 15, 30, 40, 50, 60).

6. Housse de protection (22, 32, 52, 62, 70) selon la revendication 1,
dans laquelle ledit premier anneau (28) est adapté pour s'ajuster autour d'un effecteur terminal (20) dudit système robotisé (10, 15, 30, 40, 50, 60) et ledit second anneau (24) est configuré pour s'adapter autour d'une portion de base (16) dudit système robotisé (10, 15, 30, 40, 50, 60) sans contraindre la rotation dudit bras robotisé (14).

7. Housse de protection (22, 32, 52, 62, 70) selon la revendication 6,
dans laquelle ledit premier anneau (28) s'ajuste autour dudit effecteur terminal (20) du système robotisé (10, 15, 30, 40, 50, 60) sans contraindre la rotation de l'effecteur terminal (20),
dans laquelle ledit premier anneau (28) forme de préférence un joint hermétique avec ledit système robotisé (10, 15, 30, 40, 50, 60).

8. Housse de protection (22, 32, 52, 62, 70) selon la revendication 1,
dans laquelle lesdits un ou plusieurs anneaux sectionnels (34) forment soit une connexion rigide soit une connexion non contrainte en rotation entre ladite pluralité de segments de housse (36),
ou bien lesdits un ou plusieurs anneaux sectionnels (34) sont connectés au bras robotisé (14).

9. Housse de protection (22, 32, 52, 62, 70) selon l'une quelconque des revendications 1 à 8,
dans laquelle chacun desdits segments de housse (36) est indépendamment soit réalisé en papier, en matériaux tissés, en tissus, en films de matière plastique, en feuilles ou une combinaison de ceux-ci, soit est un film de matière plastique qui est transparent.

10. Housse de protection (22, 32, 52, 62, 70) selon l'une quelconque des revendications 1 à 7,
dans laquelle le tube de protection a une longueur configurée pour encapsuler le bras robotisé (14) ; et
un tube/conduit d'irrigation, de vide ou d'air (64) est intégré audit tube de protection.

11. Housse de protection (22, 32, 52, 62, 70) selon la revendication 10, dans laquelle ledit tube/conduit d'irrigation, de vide ou d'air (64) se présente sous un motif hélicoïdal ou analogue à un ressort le long de la longueur dudit tube de protection et, de préférence, soit forme une ossature le long de la longueur dudit tube de protection, soit est préconditionné et plié avec le housse (22, 32, 52, 62, 70), où le fait de déplier le housse (22, 32, 52, 62, 70) déroule également ledit tube/conduit d'irrigation, de vide ou d'air (64) ; et dans laquelle une extrémité dudit un tube/conduit d'irrigation, de vide ou d'air (64) est positionnée au niveau de l'effecteur terminal (20) quand ledit tube de protection est déployé.

12. Système robotisé (10, 15, 30, 40, 50, 60) comprenant :
une base (16) ;
un bras robotisé (14) ayant au moins trois degrés de liberté s'étendant depuis la base (16) ; et
une housse de protection (22, 32, 52, 62, 70) selon l'une quelconque des revendications 1 à 11.

13. Procédé de déploiement d'une housse de protection (22, 32, 52, 62, 70) pour encapsuler un système robotisé (10, 15, 30, 40, 50, 60), comprenant les étapes consistant à :
maintenir un tube de la housse de protection (22, 32, 52, 62, 70) connecté à au moins un anneau d'une paire d'anneaux (24, 28) à l'avant dudit système robotisé (10, 15, 30, 40, 50, 60) ; et
déplacer ou avancer automatiquement un bras robotisé (14) dudit système robotisé (10, 15, 30, 40, 50, 60) à travers le tube de protection pour encapsuler ledit système robotisé (10, 15, 30, 40, 50, 60),
**caractérisé en ce que**
lesdits anneaux (24, 28) sont non contraints en rotation.

14. Procédé selon la revendication 13,
dans lequel ladite housse de protection (22, 32, 52, 62, 70) se déplie quand ledit bras robotisé (14) se déplace à travers un second anneau (24) de ladite paire d'anneaux (24, 28), de préférence maintenue par un utilisateur humain et/ou par un dispositif.

15. Procédé selon la revendication 14,
dans lequel le tube de protection est connecté à un premier anneau (28) de ladite paire d'anneaux (24, 28), le premier anneau (28) étant fixé à une extrémité distale du système robotisé (10, 15, 30, 40, 50, 60),
dans lequel ladite paire d'anneaux (24, 28) est fixée audit tube de protection après que le tube entoure le système robotisé (10, 15, 30, 40, 50, 60), et/ou le procédé comprend l'étape consistant à créer un joint de base en attachant ledit second anneau (24) de ladite paire d'anneaux (24, 28) à une base (16) du système robotisé (10, 15, 30, 40, 50, 60).
